(19) Europäisches Patentamt
European Patent Office
Office européen des brevets

(11) Publication number: **0 104 780**
**B1**

(12) # EUROPEAN PATENT SPECIFICATION

(45) Date of publication of patent specification: **26.11.86**

(51) Int. Cl.$^4$: **C 12 Q 1/40**

(21) Application number: **83304967.9**

(22) Date of filing: **26.08.83**

(54) **Measurement of alpha-amylase activity.**

(30) Priority: **27.08.82 JP 148756/82**
**27.07.83 JP 137311/83**

(43) Date of publication of application:
**04.04.84 Bulletin 84/14**

(45) Publication of the grant of the patent:
**26.11.86 Bulletin 86/48**

(84) Designated Contracting States:
**AT BE CH DE FR GB IT LI LU NL SE**

(56) References cited:
**EP-A-0 005 867**
**EP-A-0 034 692**
**US-A-4 321 364**

**CHEMICAL ABSTRACTS, vol. 97, no. 1, July 5, 1982, page 267, abstract no. 2605c,**

**The file contains technical information submitted after the application was filed and not included in this specification**

(73) Proprietor: **WAKO PURE CHEMICAL INDUSTRIES, LTD.**
**10, Doshomachi-3-chome**
**Higashi-ku Osaka (JP)**

(72) Inventor: **Miyashita, Yoshinobu**
**14-17 Takadono-7-chome**
**Asahi-ku Osaka (JP)**
Inventor: **Hamanaka, Tadashi**
**52-6-506, Tsuchiyamacho**
**Nada-ku Kobe (JP)**
Inventor: **Satomura, Shinji**
**Urban-Haitsu 402-go 1-5-25, Abiko**
**Sumiyoshi-ku Osaka (JP)**

(74) Representative: **Baillie, Iain Cameron et al**
**c/o Ladas & Parry Isartorplatz 5**
**D-8000 München 2 (DE)**

(56) References cited:
**COLUMBUS, Ohio (US) K. FROEHLICH: "Kinetic determination of alpha amylase using carboxymethylated starch as a substrate"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 104 780 B1

(58) References cited:

CHEMICAL ABSTRACTS, vol. 69, no. 22, November 25, 1968, page 8264, abstract no. 88153k, COLUMBUS, Ohio (US) T. HAYASHI: "Biochemical hydrolysis of sodium carboxymethyl starch. II. High-molecular-weight hydrolyzates produced under hydrolysis with salivary amylase"

CHEMICAL ABSTRACTS, vol. 70, no. 8, February 24, 1969, page 26, abstract no. 34447m, COLUMBUS, Ohio (US) T. HAYASHI: "Biochemical hydrolysis of sodium carboxymethyl starch III. Low-molecular-weight hydrolzate obtained by treatment with salivary amylase"

CHEMICAL ABSTRACTS, vol. 90, no. 23, June 4, 1979, page 245, abstract no. 182133j, COLUMBUS, Ohio (US) J.F. KENNEDY et al.: "Characteristics of alpha-amylase K, a novel amylase from a strain of Bacullus substilis"

CHEMICAL ABSTRACTS, vol. 94, no. 13, March 30, 1981, page 341, abstract no. 98767u, COLUMBUS, Ohio (US) H. MATSUI et al.: "Evidence for a single active site on sugar beet alpha-glucosidase with maltase and glucoamylase activities"

CLINICAL CHEMISTRY, vol. 25, no. 2, February 1979, EASTON Penn. (US) L. VAN LEEUWEN: "New saccharogenic determination of alpha-amylase in serum and urine", pages 215-217

CHEMICAL ABSTRACTS, vol. 87, no. 3, July 18, 1977, page 205, abstract no. 17792d, COLUMBUS, Ohio (US) J.J. MARSHALL et al.: "A new serum alpha-amylase assay of high sensitivity"

**Description**

This invention relates to a process for measuring α-amylase activity using a special substrate and, if necessary, a special stopper, and a reagent composition used for such a process.

Measurement of α-amylase activity in a sample, particularly in saliva, pancreatic juice, blood and urine in human living body is important for diagnosis in medical science. For example, α-amylase activity in blood and urine shows a remarkable increase in the case of pancreatitis, cancer of the pancreas, and parotitis compared with normal values.

Various processes for measuring α-amylase activity have been proposed and can be divided into three groups, that is, an amyloclastic method, a chromogenic method, and a saccharogenic method.

As to the amyloclastic method, the Caraway method has been most widely used, but there are some problems in that reproducibility is not good, and the like due to inhibition of coloring of starch by iodine by co-existing protein or due to short reaction time.

As to the chromogenic method, the blue starch method is generally used, wherein there is used an insoluble substrate obtained by combining starch or amylose with a dyestuff and a soluble dyestuff produced by an enzymatic reaction is measured. This method is widely used recently, but there are many problems in that the activity as substrate is weak, the reaction system is non-uniform due to insolubility, complicated procedures are necessary and application to auto analyzers is difficult, etc.

As to the saccharogenic method, the Somogyi method is typical one, but there are problems in that a higher value is obtained due to glucose in a sample, the procedures are undesirably complicated, etc.

As mentioned above, individual methods for measuring α-amylase activity have specific defects. In addition, there are defects common to the above-mentioned methods in that variations in measured values are produced depending on the quality of starch used as substrate, and the α-amylase reaction cannot be measured as a true stoichiometrical reaction.

It is an object of this invention to provide a process for measuring α-amylase activity precisely by a rate assay or by using an auto analyzer. It is another object of this invention to provide a process for measuring α-amylase activity precisely by a fixed-time assay using a special stopper and a reagent composition used therefor.

This invention provides a process for measuring α-amylase activity comprising acting α-amylase on a substrate, followed by acting a coupling enzyme on the resulting decomposed substrate to produce glucose, and determining quantitatively the glucose produced, characterised by using as substrate a modified amylose having one carboxymethyl group or a salt thereof per 6 to 35 glucose units in amylose.

This invention also provides a process for measuring α-amylase activity using a substrate and a coupling enzyme and determining quantitatively glucose produced, characterized by using glucoamylase as coupling enzyme, a modified substrate as substrate and a reaction stopper of the formula:

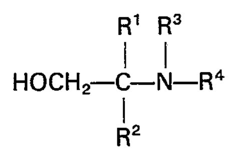

(I)

wherein $R^1$ through $R^4$ are independently hydrogen or a lower alkyl group having 1 to 4 carbon atoms, said alkyl group being able to contain a hydroxyl group, or a salt of said stopper, for stopping an enzymatic reaction caused by glucoamylase.

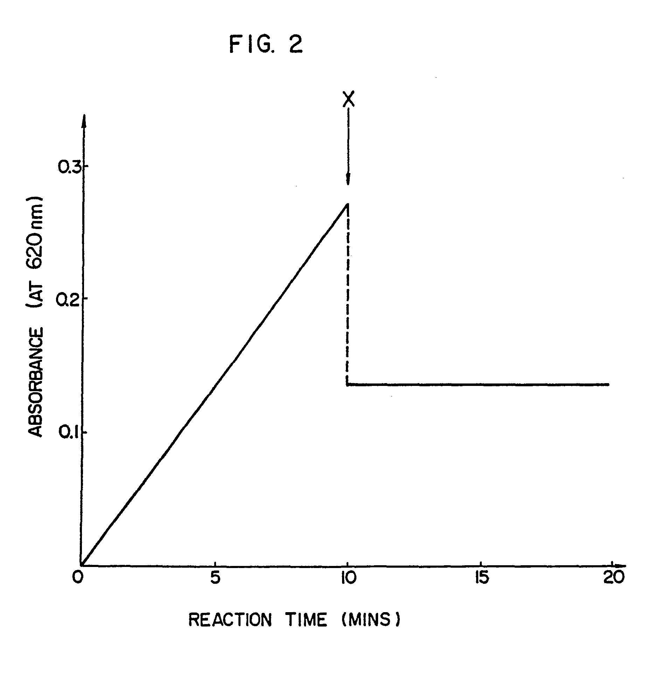

In the attached drawings, Fig. 1 shows a calibration curve for measuring α-amylase activity, and Fig. 2 shows changes of absorbance before and after addition of a stopper.

α-Amylase is an enzyme which hydrolyzes α-1,4-glycoside bonds. In starch or amylopectin having α-1,6-glycoside bonds, the α-1,6-glycoside bonds inhibit the α-amylase reaction. On the other hand, amylose is better than starch (which has been used in the carboxymethylated form—Chemical Abstracts, Vol, 97, No. 1 5 July 1982, p. 267 No. 2605c) and amylopectin as substrate since it is better in uniformity from the viewpoint of chemical structure, which results in improving measuring accuracy.

But when amylose is used as substrate in a process for measuring α-amylase activity by using glucoamylase or α-glucosidase as coupling enzyme and determining quantitatively glucose produced, there arises a defect in that glucoamylase or α-glucosidase decomposes the substrate irrespective of the α-amylase reaction, since glucoamylase or α-glucosidase is an exo type enzyme which hydrolyzes an α-1,4-glycoside bond from a non-reducing end of amylose irrespective of α-amylase. As a result, when amylose is used as substrate, there are many disadvantages in that a reagent solution for measurement cannot contain both the substrate and glucoamylase or α-glucosidase at the same time, a sufficient amount of glucoamylase or α-glucosidase for measurement cannot be used, a reagent blank value increases remarkably, it is difficult to establish the measuring method with accuracy.

But the disadvantages mentioned above are overcome by using a modified amylose having one carboxymethyl group or a salt thereof per 6 to 35 glucose units in amylose (hereinafter referred to as "modified amylose") as substrate. The salt of carboxymethyl group means one which can give a

carboxymethyl ion when dissolved. Examples of such salts of carboxymethyl group are salts of alkali metals such as Na, K, Li, etc., an ammonium salt, etc.

The modified amylose is excellent in affinity to α-amylase and can be a good substrate for α-amylase but cannot be a substrate for glucoamylase or α-glucosidase. Further, there can be obtained various advantages in that solubility for water increases remarkably, the substrate can be used in sufficient concentration suitable for the α-amylase reaction, stability in an aqueous solution is improved, and the like.

Since glucoamylase or α-glucosidase is an exo type enzyme, the modified amylose cannot be used as a substrate. In contrast, α-amylase is an endo type enzyme which hydrolyzes α-1,4-glycoside bonds in amylose at any portions, and thus the modified amylose can be used as substrate. The modified amylose is hydrolyzed by the enzymatic action of α-amylase to newly form non-reducing ends. On the newly formed non-reduced ends, α-glucosidase or glucoamylase acts to produce glucose. By determining the amount of glucose produced, there can be measured α-amylase activity. These reactions can be shown as follows:

$$\overset{\overset{X}{|}}{G}-G_m-G_n-\overset{\overset{X}{|}}{G}-G\sim \xrightarrow{\alpha\text{-amylase}} \overset{\overset{X}{|}}{G}-G_m+G_n-\overset{\overset{X}{|}}{G}-G\sim \qquad (1)$$

$$G_n-\overset{\overset{X}{|}}{G}-G\sim \xrightarrow[\alpha\text{-glucosidase}]{\text{glucoamylase or}} n\cdot G+\overset{\overset{X}{|}}{G}-G\sim \qquad (2)$$

In the above sequence, G represents a glucose unit, X is a carboxymethyl group or a salt thereof, and n and m are independently integers of 2 or more.

There have been known various methods for quantitatively determining glucose, which methods can be used in this invention. Major methods for quantitatively determining glucose are shown below.

One method is as follows. When glucose is acted by glucose oxidase, it is oxidized to produce hydrogen peroxide. The produced hydrogen peroxide oxidizes a chromogen quantitatively by means of peroxidase present to give an oxidized chromogen. The amount of glucose in the reaction solution can be measured by colorimetric determination of the color produced by the oxidized chromogen. These reactions can be shown below.

$$\text{glucose}+O_2+H_2O \xrightarrow{\text{GOD}} H_2O_2+\text{gluconic acid} \qquad (3)$$

$$H_2O_2+\underset{(\text{colorless})}{\underset{\text{chromogen}}{\text{reduced}}} \xrightarrow{\text{POD}} \underset{(\text{colored})}{\underset{\text{chromogen}}{\text{oxidized}}}+H_2O \qquad (4)$$

GOD: glucose oxidase
POD: peroxidase

Another method is as follows. Glucose is changed to glucose-6-phosphate in the presence of ATP by hexokinase. The produced glucose-6-phosphate is changed to gluconolactone-6-phosphate in the presence of NAD by glucose-6-phosphate dehydrogenase. On the other hand, NAD is reduced to NADH. By measuring an increase in absorbance of NADH at near 340 nm, the amount of glucose in the reaction solution can be determined. These reactions can be shown below.

$$\text{glucose}+\text{ATP} \xrightarrow[Mg^{2+}]{\text{hexokinase}} \text{glucose-6-phosphate}+\text{ADP} \qquad (5)$$

$$\text{glucose-6-phosphate}+\text{NAD} \xrightarrow{\text{G-6-PDH}} \text{gluconolactone-6-phosphate}+\text{NADH} \qquad (6)$$

ATP: adenosine triphosphate
ADP: adenosine diphosphate
NAD: nicotinamide adenine dinucleotide
NADH: nicotinamide adenine dinucleotide, reduced form
G-6-PDH: glucose-6-phosphate dehydrogenase

In the process of this invention wherein a sample is treated with α-amylase and simultaneously or successively with a coupling enzyme such as glucoamylase or α-glucosidase using the modified amylose as substrate and glucose produced is determined quantitatively, there is a possibility of influence of

glucose present in a sample, particularly in a living sample such as serum, urine, etc. It is an effective means to remove or extinguish the glucose present in a sample before the α-amylase reaction in practicing the process of this invention.

As to the removing or extinguishing method of glucose in the sample, there can be used a method of using glucose oxidase-catalase, a method of using hexokinase (Japanese Patent Appln Kokai (Laid-Open) No. 47495/82), a method of using glucose peroxidase, and the like, alone or in combination of these methods.

The modified amylose can be produced by reacting amylose having a molecular weight of about 3,000 to 200,000, sodium hydroxide and monochloroacetic acid in an aqueous solution. It is preferable to use 5 to 30 moles of alkali such as NaOH and 0.5 to 5 moles of monochloroacetic acid per mole of glucose unit in amylose. The reaction is preferably carried out at about 30 to 70°C for 30 minutes to 3 hours with stirring. (See the process disclosed by S. Peat et al in "Nature" vol. 159, p. 810 (1947).)

Then, the product is neutralized and then treated with glucoamylase in an aqueous solution near neutral at 37°C for 10 to 30 hours to introduce a carboxymethyl group or a salt thereof into glucose units at non-reduced ends or near to said ends for giving the modified amylose. This reaction can be shown as follows.

$$G_n{-}\overset{X}{\overset{|}{G}}{-}G_m{-}\overset{X}{\overset{|}{G}}{\sim} \xrightarrow{\text{glucoamylase}} n\cdot G+\overset{X}{\overset{|}{G}}{-}G_m{-}\overset{X}{\overset{|}{G}}{\sim} \qquad (7)$$

In the above equation, G is a glucose unit, X is a carboxymethyl group or a salt thereof, n is an integer of 1 or more, and m is an integer of about 5 to 34.

In the above-mentioned reaction, the treatment with glucoamylase is not essential, but is effective to lower the level of blank value in measured values.

Then the reaction product is subjected to dialysis using water as outer liquid. By the dialysis, by-products such as sodium chloride, sodium hydroxyacetate, etc., and glucose obtained by hydrolysis by glucoamylase are removed. After condensation, there can be obtained dried modified amylose. Degree of carboxymethylation of sodium salt of carboxymethylamylose can be obtained by a pH titration.

The number of modifying group, i.e. the carboxymethyl group or a salt thereof which is introduced into amylose, is a very important factor from the viewpoint of precision and accuracy of α-amylase activity measurement. Introduction of too many modifying groups into amylose will inhibit the action of α-amylase. When one or more modifying groups per 5 glucose units are introduced, the action of α-amylase decreases, which results in lowering in sensitivity, precision and accuracy and making it impossible to use practically. On the other hand, introduction of too small modifying groups into amylose is not preferable, since contribution to improvement in solubility of amylose by the modifying group is little and there cannot be obtained a modified amylose having sufficient solubility.

In order to maintain a necessary substrate concentration, it is necessary to introduce one or more modifying groups per 35 glucose units.

Therefore, as the modified amylose, there are used those having one modifying group per 6 to 35 glucose units in amylose.

As a sample usable in the process of this invention, there can be used any samples containing α-amylase. Examples of specimens are blood, serum, urine, etc., as components in living body.

Glucoamylase or α-glucosidase derived from animals and microorganisms can be used in this invention but not limited thereto.

Measuring conditions for practicing this invention are as follows. The reaction temperature of the formulas (1) and (2) is not particularly limited but preferably about 25 to 40°C. The reaction time can be selected freely depending on purposes. The pH in the reaction formulas (1) and (2) is not particularly limited but preferably pH is about 6 to 8. In order to maintain the pH at a preferable value, there can be used buffers such as phosphate, Good's buffers.

In addition, there can be used an activator of α-amylase such as sodium chloride, calcium chloride, potassium chloride, etc.

As the measuring method of α-amylase activity, there can preferably be used a rate assay in which a reaction rate is measured under certain conditions. It is also possible to use a fixed-time assay using a reaction stopper but in this case it is preferable to use an improved method mentioned below. The term "fixed-time assay" means a method for measuring enzymatic activity wherein there is determined a changing amount of a substrate from an addition of a stopper to a reaction solution after a prescribed reaction time to a time at which the reaction is stopped by said stopper.

By using the modified amylose as substrate for measuring α-amylase activity, there can establish measuring methods having the following advantages:

(i) Since the modified amylose is easily soluble in water, there can be used any substrate concentrations sufficient for the α-amylase reaction. Thus, there can be obtained good calibration relationship and wide measuring ranges.

(ii) Since the modified amylose cannot be a substrate for glucoamylase and α-glucosidase and can specifically be a substrate for α-amylase, no side reaction takes place and reagent blank values are extremely small.

(iii) Since a necessary and sufficient amount of glucoamylase and α-glucosidase can be used, the reactions after the α-amylase reaction are fast and accurate rate assay becomes possible.

(iv) Thus, the glucose produced by the above mentioned reactions can be determined quantitatively by a conventional method with high sensitivity and good precision.

(v) Measuring procedures are simple.

(vi) Application to auto analyzers is easy.

(vii) Reagent solutions for measurement are stable.

In order to measure the α-amylase activity by the fixed-time assay (or one point assay) effectively, it is necessary to use a special reaction stopper which can stop effectively the reaction using the modified substrate.

Such a stopper is represented by the following formula (I) or a salt thereof.

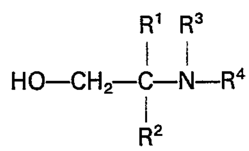

(I)

In the above formula (I), $R^1$ through $R^4$ are independently hydrogen or a lower alkyl group having 1 to 4 carbon atoms, said alkyl group being able to contain a hydroxyl group.

Since the stopper of the formula (I) or a salt thereof can effectively stop the activity of glucoamylase, the α-amylase activity can be measured quantitatively with high accuracy and good reproducibility.

It has been reported that tris(hydroxymethyl)aminomethane has an inhibition action against the activity of glucoamylase. For example, according to an article recited in Agr. Biol. Chem., *41,* 2149—2161 (1977), particularly tris(hydroxymethyl)aminomethane has an inhibitory action against the activity of glucoamylase. When 0.39 unit of glucoamylase derived from *Aspergillus awamori* and 50 mM of tris(hydroxymethyl)aminomethane are heated at pH 5.3 at 37°C for 5 minutes, followed by addition of soluble starch (0.2%) to measure residual activity from the amount of glucose produced, the residual activity is found to be 49.9%. According to another article recited in Agr. Biol. Chem., *41,* 2139—2148, (1977), glucoamylase derived from *Mucor rouxianns* is inhibited its activity by tris(hydroxymethyl)aminomethane in an amount of 5 mM at pH 5.3. The residual activity is found to be 26.7% or 28.8%.

As mentioned above, the inhibitory action of tris(hydroxymethyl)aminomethane against the activity of glucoamylase is incomplete and considerable residual activity is admitted. On the other hand, in the fixed-time assay wherein an enzymatic reaction in the proceeding reaction is stopped by a stopper, the stopper is required to stop the enzyme activity almost completely in a short time in order to give measurement results with high accuracy and good reproducibility. Therefore, it is concluded that tris(hydroxymethyl)aminomethane is not suitable for use as a stopper for glucoamylase used in a proceeding reaction due to incomplete inhibitory action.

But the present inventors have noticed the quality of substrate used and found that the inhibitory action of a stopper against an enzyme is competitive inhibition with a substrate used therein. Thus, properly selecting a substrate, tris(hydroxymethyl)aminomethane can stop the activity of glucoamylase effectively. The same effect can be shown in the compounds of the formula (I) or salts thereof.

Examples of the reaction stoppers represented by the formula (I) are as follows:

(i) Tris(hydroxymethyl)aminomethane (2-amino-2-hydroxymethyl-1,3-propanediol)

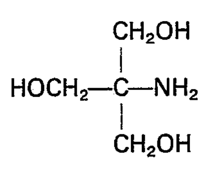

(ii) 2-Amino-1,3-propanediol

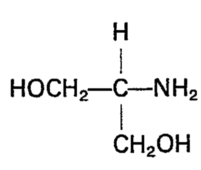

(iii) 2-Amino-2-methyl-1,3-propanediol

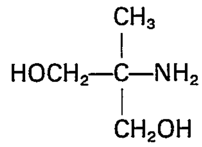

(iv) 2-Amino-2-ethyl-1,3-propanediol

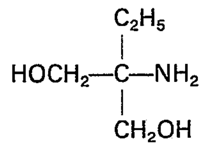

(v) 2-Amino-1,4-butanediol

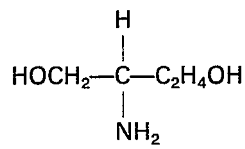

(vi) Monoethanolamine

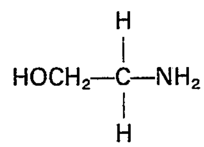

(vii) Diethanolamine

$NH(C_2H_4OH)_2$

(viii) Triethanolamine

$N(C_2H_4OH)_3$

Examples of salts of the compounds of the formula (I) are salts of mineral acids such as hydrochloric acid, sulfuric acid, etc., or salts of organic acids such as maleic acid, oxalic acid, etc., of the compounds of the formula (I).

As the special substrate used together with stopper of the formula (I) or a salt thereof, there can be used the modified amylose having one carboxymethyl group or a salt thereof per 6 to 35 glucose units in amylose as mentioned above. This substrate is particularly preferable from the viewpoint of solubility.

There can also be used as substrate modified amylose having 2-pyridylamino groups, 3-pyridylamino groups, anilino groups, 2-hydroxyanilino groups, methylanilino groups, carboxyphenylamino groups or the like modifying groups other than the carboxymethyl groups, or polysaccharide having modifying groups at non-reducing ends in an amount suitable as substrate for α-amylase, e.g., sodium starch glycolate (carboxymethylated starch) (Clinica Chimica Acta, *76,* 277—283 (1977)).

When polysaccharide having α-1,4-glycoside bonds such as starch or amylose is used as substrate, glucoamylase decomposes the substrate irrespective of the α-amylase reaction in the method for measuring the glucose produced by the enzymatic actions of α-amylase and glucoamylase used as coupling enzyme, since glycoamylase is an exo type enzyme which hydrolyzes α-1,4-glycoside bonds from non-reducing ends of amylose irrespective of α-amylase. Thus, starch and amylose conventionally used cannot be used as substrate.

Measuring conditions for carrying out the fixed-time assay are as follows. Particularly effective concentration of the stopper of the formula (I) or a salt thereof at the time of reaction stoppage is 50 mM or more, preferably 100 mM or more up to its solubility limit.

The reaction temperature of the α-amylase reaction (1) and the coupling enzyme reaction (2) is not limited particularly but preferably about 25 to 40°C. The reaction time can be selected freely depending on purposes. The pH in the reaction formula (1) or (2) is not particularly limited but preferably pH is about 6 to 8. In order to maintain the pH at a preferable value, there can be used buffers such as phosphate, Good's buffers. Further, pH of the final liquid is preferably 6.5 to 8.5, although slightly changes depending on the glucose determination system.

By the use of a special stopper of the formula (I) or a salt thereof in combination with a special modified substrate, the use of a special auto analyzer is not necessary, a manual method can be used and measuring methods practically useful are widened, and a large number of specimens can be measured at one time by a manual method. Further, since the reaction can be stopped extremely rapidly, measuring error is small and accurate values can be obtained with good reproducibility. In addition, since a strong acid or strong alkali is not necessary and a mild liquid with near neutral can be used, it is easy to maintain stable conditions for color formation.

This invention is illustrated by way of the following Examples, in which all percents are by weight unless otherwise specified.

Example 1

Preparation of modified amylose

Reagents

(1) Amylose

Amylose having an average molecular weight of about 150,000.

(2) Sodium hydroxide solution

A 50% sodium hydroxide solution is prepared.

(3) Monochloroacetic acid solution

A 25% monochloroacetic acid solution is prepared.

(4) Glucoamylase

Glucoamylase derived from *Rhizopus.*

Experimental method

To 10 g of amylose, 150 ml of water and 50 ml of the sodium hydroxide solution are added and mixed at 50°C for 1 hour with stirring. Then, 40 ml of the 25% monochloroacetic acid solution is added thereto dropwise and heated at 50°C for additional 1 hour. After cooling to room temperature, the resulting solution is neutralized with hydrochloric acid. Subsequently, 1000 units of glucoamylase is added thereto and incubated at 37°C for about 15 hours.

In order to remove by-produced sodium hydroxide, sodium hydroxyacetate and glucose, the reaction mixture is placed in a dialysis tube and subjected to dialysis using deionized water as outer liquid for 50 hours.

After condensing the resulting solution, the desired modified amylose is obtained by freeze-drying. The yield is 90%.

The degree of carboxymethylation of amylose is obtained by adding 0.1 N hydrochloric acid to a prescribed amount of the resulting modified amylose and conducting a pH titration using 0.1 N sodium hydroxide. As a result, it is confirmed that one sodium carboxymethyl group per about 22 glucose units is introduced into the amylose.

Example 2

Reagent

(1) Reagent solution (1)

In purified water, 40 mmole of Good's buffer solution (PIPES), 50,000 units of glucoamylase, 100,000 units of glucose oxidase, 200 units of mutarotase, 500,000 units of catalase, 0.7 mmole of 4-aminoantipyrine, 15 mmole of sodium chloride and 5 mmole of calcium chloride are dissolved. The resulting solution is made pH 6.9 with sodium hydroxide and the whole amount is made 1 liter.

(2) Reagent solution (2)

In purified water, 40 mmole of Good's buffer solution (PIPES), 15,000 units of peroxidase, 15 mmole of sodium chloride, 5 mmole of calcium chloride, 3 g of modified amylose obtained in Example 1, 15 mmole of sodium azide and 10 mmole of phenol are dissolved. The resulting solution is made pH 6.9 with sodium hydroxide and the whole amount is made 1 liter.

Measuring method

To 2 ml of the reagent solution (1), 20 µl of serum specimen is added and incubated at 37°C for 5 minutes. To this, 1 ml of the reagent solution (2) is added and reacted at 37°C. Change of absorbance (ΔE/min) at 505 nm is measured for 3 minutes from after 2 minutes to 5 minutes.

On the other hand, a calibration curve is prepared by using standard specimens having known α-amylase activity and the same procedures as mentioned above. Using this calibration curve, α-amylase activity of the test specimen can be obtained.

The relationship between the α-amylase activity (Somogyi unit/dl) of standard specimens and the

change of absorbance at 505 nm (ΔE/min) is shown in Fig. 1. The α-amylase activity of standard specimen is 500 Somogyi units.

Example 3
Reagents
(1) Reagent solution (3)
The same as the reagent solution (1) used in Example 2.

(2) Reagent solution (4)
The same as the reagent solution (2) used in Example 2.

(3) Reagent solution (5)
The same as the reagent solution (2) used in Example 2 except for using 0.3 g of amylose having an average molecular weight of 16,000 in place of 3 g of the modified amylose having sodium carboxymethyl group.

(4) Serum specimen
Serum containing α-amylase having activity of 300 Somogyi units.

Measuring method
To 2 ml of the reagent solution (3), 20 µl of serum specimen is added. On the other hand, to 2 ml of the reagent solution (3), 20 µl of purified water is added. These specimens are incubated at 37°C for 5 minutes. To each specimen, 1 ml of the reagent solution (4) is added and reacted at 37°C. Change of absorbance (ΔE/min) at 505 nm is measured for 3 minutes from after 2 minutes to 5 minutes. These results are measured values of the serum specimen and reagent blank using the modified amylose as substrate and shown in Table 1.

On the other hand, using the reagent solution (5) in place of the reagent solution (4), measured values are obtained in the same manner as mentioned above and the results are shown in Table 1.

TABLE 1

| | Change of absorbance during 3 minutes (ΔE/min) | |
|---|---|---|
| Substrate | Blank | Specimen |
| Modified amylose | 0.006 | 0.372 |
| Amylose (non-modified) | 0.030 | 0.114 |

Example 4
Reagent
(1) Reagent solution (6)
In purified water, 10 mmole of Good's buffer solution (PIPES), 20,000 units of glucoamylase, 100,000 units of glucose oxidase, 200 units of mutarotase, 500,000 units of catalase, 1 mmole of N,N-diethylxylidine, 15 mmole of sodium chloride, and 5 mmole of calcium chloride are dissolved. The resulting solution is made pH 6.9 with sodium hydroxide and the whole amount is made 1 liter.

(2) Reagent solution (7)
In purified water, 20 mmole of Good's buffer solution (PIPES), 5,000 units of peroxidase, 15 mmole of sodium chloride, 6 g of carboxymethylamylose prepared in Example 1, 15 mmole of sodium azide, and 0.5 mmole of 4-aminoantipyrine are dissolved. The resulting solution is made pH 6.9 with sodium hydroxide and the whole amount is made 1 liter.

(3) Reaction stopper solution (1)
In purified water, 0.6 mole of tris(hydroxymethyl)aminomethane is dissolved and the pH is made 7.5 with HCl, while making the whole amount 1 liter.

Measuring method
To 1 ml of the reagent solution (6), 10 µl of serum specimen is added and incubated at 37°C for 5 minutes. To this, 1 ml of the reagent solution (7) is added and reacted at 37°C for 10 minutes. To the resulting solution, the reaction stopper solution (1) is added in an amount of 2 ml to stop the reaction and absorbance at 620 nm is measured.

On the other hand, a calibration curve is prepared by using standard specimens having known

α-amylase activity and the same procedures as mentioned above. Using this calibration curve, α-amylase activity of the test specimen can be obtained.

Fig. 2 shows changes of absorbance before and after the addition of the reaction stopper solution (X) to a serum specimen containing α-amylase having an activity of 80 Somogyi unit/dl. As shown in Fig. 2, the absorbance becomes constant after the addition of the reaction stopper solution.

Example 5

Reagent

(1) Reagent solution (8)

In purified water, 20 mmole of potassium phosphate, 30,000 units of glucoamylase, 10,000 units of hexokinase, 10,000 units of glucose-6-phosphate dehydrogenase, 5 mmole of magnesium chloride, 2 mmole of NAD (nicotinamide adenine dinucleotide), 2 mmole of ATP (adenosine triphosphate), and 3 g of carboxymethylamylose are dissolved. The resulting solution is made pH 7.0 with KOH and the whole amount is made 1 liter.

(2) Reaction stopper solution (2)

In purified water, 0.4 mole of tris(hydroxylmethyl)aminomethane is dissolved and the pH is made 7.0 with HCl, while making the whole amount 1 liter.

Measuring method

To 2 ml of the reagent solution (8), 20 µl of serum specimen is added and incubated at 37°C for 10 minutes. To this, 1 ml of the reaction stopper solution (2) is added to stop the reaction and absorbance at 340 nm is measured.

As specimen blank, a solution is prepared in the same manner as described in the reagent solution (8) except for not using carboxymethylamylose. Using said solution, absorbance at 340 nm is measured in the same manner as mentioned above.

On the other hand, a calibration curve is prepared by using standard specimens having known α-amylase activity and the same procedures as mentioned above, while removing the specimen blank value therefrom. Using this calibration curve, α-amylase activity of the test specimen is obtained.

Example 6

Reagent

(1) Reagent solution (9)

In purified water, 10 mmole of Good's buffer solution (PIPES), 20,000 units of glucoamylase, 100,000 units of glucose oxidase, 100 units of mutarotase, 500,000 units of catalase, 1 mmole of N,N-diethylxylidine, 15 mmole of sodium chloride and 5 mmole of calcium chloride are dissolved. The resulting solution is made pH 6.9 with NaOH and the whole amount is made 1 liter.

(2) Reagent solution (10)

In purified water, 20 mmole of Good's buffer solution (PIPES), 5,000 units of peroxidase, 15 mmole of sodium chloride, 6 g of carboxymethylamylose prepared in Example 1, 15 mmole of sodium azide and 0.5 mmole of 4-aminoantipyrine are dissolved. The resulting solution is made pH 6.9 with NaOH and the whole amount is made 1 liter.

(3) Reaction stopper solution (3)

In purified water, 0.6 mole (36 g) of monoethanolamine is dissolved and the pH is made 7.5, while making the whole amount 1 liter.

Measuring method

To 1 ml of the reagent solution (9), 10 µl of serum specimen is added and incubated at 37°C for 5 minutes. To this, 1 ml of the reagent solution (10) is added and reacted at 37°C for 10 minutes. To the resulting solution, the reaction stopper solution (3) is added in an amount of 2 ml to stop the reaction. Then, absorbance at 620 nm is measured.

On the other hand, a calibration curve is prepared by using standard specimens having known α-amylase activity and the same procedures as mentioned above. Using this calibration curve, α-amylase activity of the test specimen can be obtained.

**Claims**

1. A process for measuring α-amylase activity comprising reacting α-amylase with a substrate, followed by reacting a coupling enzyme with the resulting decomposed substrate to produce glucose, and determining quantitatively the glucose produced, characterised in that the substrate is a modified amylose having one carboxymethyl group or a salt thereof per 6 to 35 glucose units in amylose.

2. A process according to Claim 1, wherein the coupling enzyme is glucoamylase or α-glucosidase.

3. A process according to Claim 1, wherein the salt of carboxymethyl group is an alkali metal salt or ammonium salt of carboxymethyl group.

4. A process according to Claim 1, wherein the α-amylase activity is measured by a rate assay.

5. A process according to Claim 1, wherein the coupling enzyme is glucoamylase and the α-amylase activity is measured by a fixed-time assay using a reaction stopper of the formula:

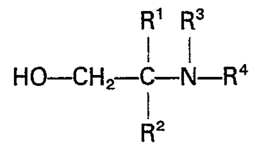 (I)

wherein $R^1$ through $R^4$ are independently hydrogen or a lower alkyl group having 1 to 4 carbon atoms, said alkyl group being able to contain a hydroxyl group, or a salt of said stopper for stopping the enzymatic reaction caused by glucoamylase.

6. A process according to Claim 5, wherein the concentration of reaction stopper at the time of reaction stoppage is 50 mM or more.

7. A process according to Claim 5, wherein the reaction stopper of the formula (I) is 2-amino-2-hydroxymethyl-1,3-propanediol, 2-amino-1,3-propanediol, 2-amino-2-methyl-1,3-propanediol, 2-amino-2-ethyl-1,3-propanediol, 2-amino-1,4-butanediol, monoethanolamine, diethanolamine, or triethanolamine.

8. A process according to Claim 5, wherein the salt of the reaction stopper of the formula (I) is a mineral acid salt or organic acid salt of the reaction stopper of the formula (I).

9. A combination of reagents for measuring α-amylase activity by a rate assay, comprising a modified linear amylose, having one carboxymethyl group or a salt thereof per 6 to 35 glucose units in the amylose, as substrate and a coupling enzyme.

10. A combination of reagents for measuring α-amylase activity by a fixed-time assay, comprising a modified amylose having one carboxymethyl group or a salt thereof per 6 to 35 glucose units in the amylose, as substrate, glucoamylase as a coupling enzyme and a reaction stopper of the formula:

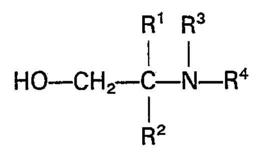 (I)

wherein $R^1$ through $R^4$ are independently hydrogen or a lower alkyl group having 1 to 4 carbon atoms, said alkyl group being able to contain a hydroxyl group, or a salt of said stopper.

**Patentansprüche**

1. Verfahren zum Messen der α-Amylaseaktivität, in den α-Amylase mit einem Substrat umgesetzt und dann mit dem so erhaltenen zersetzten Substrat ein Koppelungsenzym unter Bildung von Glucose umgesetzt und die erzeugte Glucose quantitativ bestimmt wird, dadurch gekennzeichnet, daß das substrat eine modifizierte lineare Amylose ist, die pro 6 bis 35 Glucoseeinheiten der Amylose eine Carboxymethylgruppe oder ein davon abgeleitetes Salz enthält.

2. Verfahren nach Anspruch 1, dadurch gekennzeichnet, daß das Koppelungsenzyl Glucoamylase oder α-Glucosidase ist.

3. Verfahren nach Anspruch 1, dadurch gekennzeichnet, daß das Salz der Carboxymethylgruppe ein Alkalimetallsalz oder Ammoniumsalz der Carboxymethylgruppe ist.

4. Verfahren nach Anspruch 1, dadurch gekennzeichnet, daß die α-Amylaseaktivität durch Bestimmung einer Menge pro Zeiteinheit gemessen wird.

5. Verfahren nach Anspruch 1, dadurch gekennzeichnet, daß das Kopplungsenzym Glucoamylase ist und die α-Amylaseaktivität durch Bestimmung während einer bestimmten Zeit mit Hilfe einer Reaktionsabbruchverbindung der Formel

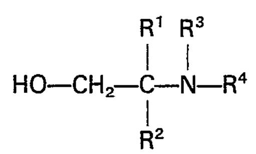 (I)

gemessen wird, in der $R^1$ bis $R^4$ unabhängig voneinander Wasserstoff oder eine niedere Alkylgruppe mit 1 bis 4 Kohlenstoffatomen sind, wobei die niedere Alkylgruppe eine Hydroxylgruppe enthalten kann, oder

mit Hilfe eines Salzes der Reaktionsabbruchverbindung, um einen Abbruch der durch die Glucoamylase verursachten enzymatischen Reaktion zu bewirken.

6. Verfahren nach Anspruch 5, dadurch gekennzeichnet, daß die Konzentration der Reaktionsabbruchverbindung im Zeitpunkt des Reaktionsabbruches 50 mM oder mehr beträgt.

7. Verfahren nach Anspruch 5, dadurch gekennzeichnet, daß die Reaktionsabbruchverbindung der Formel (I) 2-Amino-2-hydroxymethyl-1,3-propandiol, 2-Amino-1,3-propandiol, 2-Amino-2-methyl-1,3-propandiol, 2-Amino-2-ethyl-1,3-propandiol, 2-Amino-1,4-butandiol, Monoethanolamin, Diethanolamin oder Triethanolamin ist.

8. Verfahren nach Anspruch 5, dadurch gekennzeichnet, daß das Salz der Reaktionsabbruchverbindung der Formel (I) ein Salz einer Mineralsäure oder einer organischen Säure mit der Reaktionsabbruchverbindung der Formel (I) ist.

9. Kombination von Reagenzien zum Messen der α-Amylaseaktivität durch Bestimmung einer Menge pro Zeiteinheit, mit einem Substrat aus einer modifizierten linearen Amylose, die pro 6 bis 35 Glucoseeinheiten der Amylose eine Carboxymethylgruppe oder ein von dieser abgeleitetes Salz enthält, und einem Koppelungsenzym.

10. Kombination von Reagenzien zum Messen der α-Amylaseaktivität durch eine Bestimmung während einer bestimmten Zeit, mit einem Substrat aus einer modifizierten linearen Amylose, die pro 6 bis 35 Glucoseeinheiten der Amylose eine Carboxymethylgruppe oder ein von dieser abgeleitetes Salz enthält, ferner Glucoamylase als Koppelungsenzym und einer Reaktionsabbruchverbindung der Formel

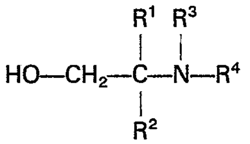

(I)

in der $R^1$ bis $R^4$ unabhängig voneinander Wasserstoff oder eine niedere Alkylgruppe mit 1 bis 4 Kohlenstoffatomen sind, wobei die niedere Alkylgruppe eine Hydroxylgruppe enthalten kann, oder ein Salz der Reaktionsabbruchverbindung.

**Revendications**

1. Procédé de mesure de l'activité de l'α-amylase consistant à faire réagir l'α-amylase avec un substrat, puis à faire réagir une enzyme de couplage avec le substrat décomposé obtenu pour produire du glucose, et à déterminer quantitativement le glucose produit, caractérisé en ce que le substrat est un amylose modifié ayant un groupe carboxyméthyle ou un de ses sels pour 6 à 35 motifs glucose dans l'amylose.

2. Procédé suivant la revendication 1, dans lequel l'enzyme de couplage est la glucoamylase ou l'α-glucosidase.

3. Procédé suivant la revendication 1, dans lequel le sel du groupe carboxyméthyle est un sel de métal alcalin ou un sel d'ammonium du groupe carboxyméthyle.

4. Procédé suivant la revendication 1, dans lequel l'activité de l'α-amylase est mesurée par une mesure de vitesse.

5. Procédé suivant la revendication 1, dans lequel l'enzyme de couplage est la glucoamylase et l'activité de l'α-amylase est mesurée par un essai à temps fixé en utilisant un agent d'arrêt de la réaction répondant à la formule:

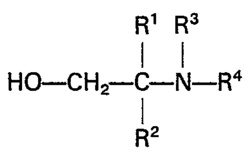

(I)

dans laquelle $R^1$ à $R^4$ sont indépendamment l'hydrogène ou un groupe alkyle inférieur en $C_1$ à $C_4$, ce groupe alkyle étant susceptible de contenir un groupe hydroxyle, ou un sel de cet agent d'arrêt pour arrêter la réaction enzymatique causée par la glucoamylase.

6. Procédé suivant la revendication 5, dans lequel la concentration de l'agent d'arrêt de la réaction au moment de l'arrêt de la réaction est de 50 mM ou davantage.

7. Procédé suivant la revendication 5, dans lequel le sel de l'agent d'arrêt de la réaction répondant à la formule (I) est le 2-amino-2-hydroxyméthyl-1,3-propanediol, le 2-amino-1,3-propanediol, le 2-amino-2-méthyl-1,3-propanediol, le 2-amino-2-éthyl-1,3-propanediol, le 2-amino-1,4-butanediol, la monoéthanolamine, la diéthanolamine, ou la triethanolamine.

8. Procédé suivant la revendication 5, dans lequel le sel de l'agent d'arrêt de la réaction répondant à la formule (I) est un sel d'acide minéral ou un sel d'acide organique de l'agent d'arrêt de la réaction répondant à la formule (I).

9. Combinaison de réactifs pour mesurer l'activité de l'α-amylase par une mesure de vitesse,

comprenant un amylose linéaire modifié, ayant un groupe carboxyméthyle ou un sel de celui-ci pour 6 à 35 motifs glucose dans l'amylose, comme substrat, et une enzyme de couplage.

10. Combinaison de réactifs pour mesurer l'activité de l'α-amylase par un essai à temps fixé, comprenant un amylose modifié ayant un groupe carboxyméthyle ou un sel de celui-ci pour 6 à 35 motifs glucose dans l'amylose comme substrat, de la glucoamylase comme enzyme de couplage et un agent d'arrêt de la réaction répondant à la formule

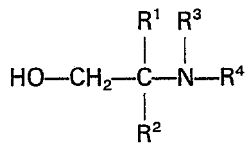

(I)

dans laquelle $R^1$ à $R^4$ sont indépendamment l'hydrogène ou un groupe alkyle inférieur en $C_1$ à $C_4$, ce groupe alkyle étant susceptible de contenir un groupe hydroxyle, ou un sel de cet agent d'arrêt.

FIG. 1

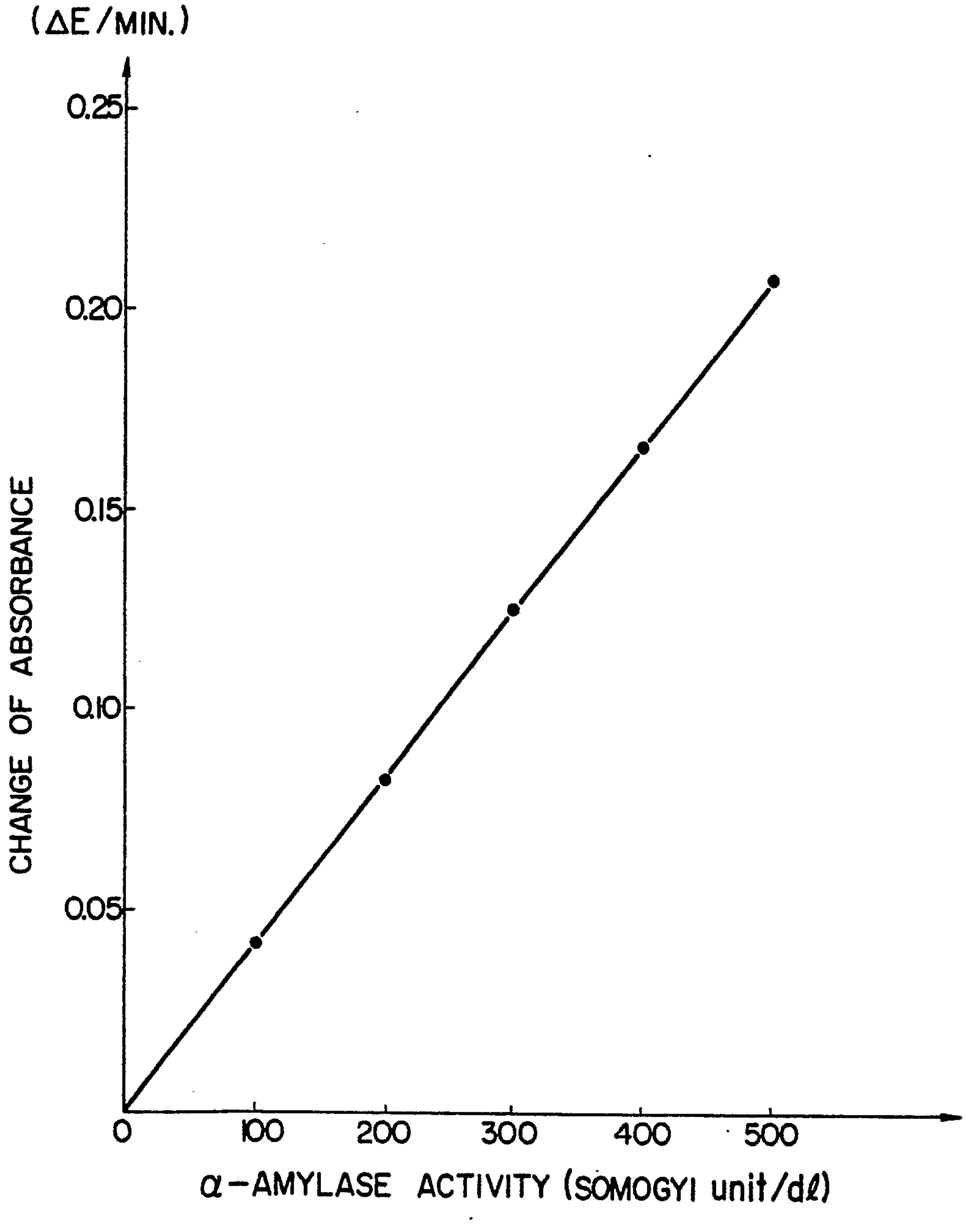
(ΔE/MIN.)
0.25
0.20
0.15
0.10
0.05
CHANGE OF ABSORBANCE
0
100
200
300
400
500
α-AMYLASE ACTIVITY (SOMOGYI unit/dℓ)

FIG. 2